# EUROPÄISCHE PATENTSCHRIFT

(11) **EP 2 004 589 B1**
(45) Veröffentlichungstag und Bekanntmachung des Hinweises auf die Patenterteilung: **12.04.2017**
(21) Anmeldenummer: 07704355.2
(22) Anmeldetag: 05.02.2007
(51) Int. Cl.: C07C 51/41, C07C 63/307, C07F 3/00, B01J 20/26

(54) **VERFAHREN ZUR HERSTELLUNG PORÖSER METALLORGANISCHER GERÜSTMATERIALIEN**
PROCESS FOR PREPARING POROUS METAL-ORGANIC FRAMEWORK MATERIALS
PROCEDE DE FABRICATION D'UN MATERIAU DE CHASSIS METALLO-ORGANIQUE POREUX

(30) Priorität: 10.02.2006 EP 06101533
(43) Veröffentlichungstag der Anmeldung: 24.12.2008
(73) Patentinhaber: BASF SE, 67056 Ludwigshafen am Rhein (DE)
(72) Erfinder: SCHUBERT, Markus, 67063 Ludwigshafen (DE); MÜLLER, Ulrich, 67435 Neustadt (DE); HESSE, Michael, 67549 Worms (DE); DIEHLMANN, Uwe, 67454 Hassloch (DE)
(86) Internationale Anmeldenummer: PCT/EP2007/051071
(87) Internationale Veröffentlichungsnummer: WO 2007/090809

(56) Entgegenhaltungen:
- EP-A2- 1 070 538
- WO-A1-02/088148

## Beschreibung

Die vorliegende Erfindung betrifft Verfahren zur Herstellung poröser metallorganischer Gerüstmaterialien.

Poröse metallorganische Gerüstmaterialien bilden eine interessante Substanzklasse, die für verschiedenste Anwendungen eine Alternative zu anorganischen Zeolithen sein können.

Solche Anwendungen liegen zum Beispiel auf dem Gebiet der Speicherung, Abtrennung oder kontrollierten Abgabe chemischer Stoffe, wie beispielsweise von Gasen, oder auf dem Gebiet der Katalyse. Hierbei spielt insbesondere die Porosität des organischen Gerüstmaterials eine ausschlaggebende Rolle. Durch die im metallorganischen Gerüstmaterial in definierter Form vorhandenen Poren wird zum einen die spezifische Oberfläche des Materials erhöht und eine selektive Abtrennung von Gemischen ermöglicht. Gleiches gilt für solche Materialien, wenn diese bei chemischen Umsetzungen, beispielsweise bei katalytischen Reaktionen, als Trägermaterial eingesetzt werden.

Metallorganische Gerüstmaterialien sind im Stand der Technik bekannt und enthalten typischerweise mindestens eine an mindestens ein Metallion koordinativ gebundene mindestens zweizähnige organische Verbindung. Für solche Gerüstmaterialien wird häufig die Abkürzung MOF (Englisch für "metal organic framework") verwendet.

Poröse metallorganische Gerüstmaterialien weisen analog zu organischen Polymeren ein unendliches Gerüst auf, welches durch wiederholende Einheiten aufgebaut ist.

Es existiert jedoch darüber hinaus auch eine Gruppe metallorganischer Gerüstmaterialien, welche in der jüngsten Literatur als so genannte "beschränkte" Gerüstmaterialien beschrieben werden. Durch spezielle Wahl der organischen Verbindung erstreckt sich das Gerüst nicht unendlich. Es findet vielmehr eine Ausbildung von Polyedern statt. A. C. Sodic et al., J. Am. Chem. Soc. 127 (2005), 7110-7118, beschreiben beispielsweise solche speziellen Gerüstmaterialien. Hierbei werden diese zur Abgrenzung gegenüber polymeren MOF-Materialien als metallorganische Polyeder (MOP = metal organic polyhedra) genannt. All diesen metallorganischen Gerüstmaterialien ist deren Porosität gemein. Eng verknüpft mit der Porosität solcher Materialien ist deren spezifische Oberfläche, welche deren Eigenschaften stark beeinflusst. Als ein Maß für die Charakterisierung solcher Oberflächen ist die spezifische Oberfläche nach Langmuir anzusehen.

Es ist daher bei der Herstellung solcher Materialien neben einer guten Ausbeute auch die Erzeugung hoher spezifischer Oberflächen bei der Herstellung von großer Bedeutung. Dies gilt insbesondere bei der Herstellung großer Mengen an Gerüstmaterial.

WO 02/088148 A1 offenbart zinkbasierte, isoretikuläre metallorganische Gerüstmaterialien sowie Verfahren zu deren Herstellung.

Eine besondere Gruppe metallorganischer Gerüstmaterialien stellen die Kupferorganischen Gerüstmaterialien dar. Es sind beispielsweise für Cu-organische Gerüstmaterialien, bei denen das Metall ein Kupfer(II)-Ion ist und die organische Verbindung 1,3,5-Benzoltricarbonsäure darstellt, zahlreiche Vorschriften in der Literatur beschrieben. Darüber hinaus wurden neue Cu-organische Gerüstmaterialien auf elektrochemischem Weg - wie in WO-A 2005/049892 beschrieben - hergestellt.

Q. M. Wang et al., Microporous and Mesoporous Materials 55 (2002), 217-230 beschreiben die Herstellung von Kupfer(II)-benzol-1,3,5-tricarboxylat (Cu-BTC) in einem Ethanol/Wasser-Gemisch unter Verwendung von Kupfernitrathydrat im Autoclav. Hierbei werden spezifische Oberflächen von unter 1000 m²/g erhalten.

Auch die darin beschriebenen Versuche zur Optimierung in wässrigen Lösemittelsystemen unter Hydrothermalbedingungen sowie unter Rückfluss ergeben lediglich spezifische Oberflächen von etwas über 1000 m²/g.

K. Schlichte et al., Microporous and Mesoporous Materials 73 (2004), 81-88 beschreiben die Herstellung von Cu-BTC in einem Wasser/Ethanol-Gemisch unter Verwendung von Kupfernitrathydrat unter hydrothermalen Bedingungen. Ein Scale-up hat ergeben, dass eine ideale Temperatur für Hydrothermalbedingungen bei etwa 120 °C liegt.

Ein Ethanol/Wasser-Gemisch wird auch bei S. S.-Y. Chui et al., SCIENCE 283 (1999), 1148-1150, verwendet. Hierbei werden ebenfalls spezifische Oberflächen von weniger als 1000 m²/g erhalten.

Schließlich wird in EP-A 1 070 538 Cu-BTC in Ethanol unter Hydrothermalbedingungen erhalten.

All den oben beschriebenen konventionellen Synthesen ist gemein, dass diese niedrig siedende Lösemittel oder Wassergemische als Lösemittel verwenden und diese zusammen mit den erforderlichen Reagenzien, wobei das Kupfer in Form von Kupfernitrathydrat eingesetzt wird, unter Hydrothermalbedingungen eingesetzt werden.

Die Verwendung hoher Drücke stellt jedoch an die Syntheseapparatur zur Herstellung eines porösen metallorganischen Gerüstmaterials hohe Anforderungen. Es ist üblicherweise nur eine Batch-Synthese in vergleichsweise kleinen Apparaturen möglich und beschrieben. Ein Scale-up erweist sich als sehr aufwendig.

Darüber hinaus ist die Verwendung von Nitratsalzen insbesondere bei höheren Temperaturen problematisch, da hierbei nitrose Gase, welche giftig sind, entstehen können.

Eine Aufgabe der vorliegenden Erfindung besteht somit darin, Verfahren zur Herstellung von porösen Cu-organischen Gerüstmaterialien, wie Cu-BTC, bereitzustellen, wobei die oben beschriebenen Nachteile vermieden werden und dabei Gerüstmaterialien in guter Ausbeute, in großer Menge und mit möglichst hohen spezifischen Oberflächen zu erhalten.

Die Aufgabe wird gelöst durch ein Verfahren zur Herstellung eines porösen metallorganischen Gerüstmaterials den Schritt enthaltend

Umsetzung eines Reaktionsgemisches in der Flüssigphase aus mindestens einer Kupferverbindung mit mindestens einer mindestens zweizähnigen organischen Verbindung, die koordinativ an das Kupfer binden kann, in Gegenwart eines nicht-wässrigen Lösemittels, wobei die mindestens eine mindestens zweizähnige organische Verbindung eine Tri- oder Tetracarbonsäure ist, die Umsetzung unter Atmosphärendruck in einem Bereich von 90°C bis 150 °C erfolgt, die Kupferverbindung eine andere als Kupfer(II)-nitrat ist und wobei das nicht-wässrige Lösemittel ausgewählt ist aus der Gruppe bestehend aus C₄₋₃₀-Alkanol, Dimethylsulfoxid (DMSO), N,N-Dimethylformamid (DMF), N,N-Diethylformamid (DEF), N,N-Dimethylacetamid (DMAc), Acetonitril, Toluol, Dioxan, Chlorbenzol, Methylethylketon (MEK), Pyridin, gegebenenfalls halogeniertes C₇₋₂₀₀-Alkan, Sulfolan, Alkylenpolyole, wie Ethylenglykol, Polyalkylenpolyole, wie Polyethylenglykol, Glycerin, N-Methylpyrrolidon (NMP), gamma-Butyrolacton, alicyclische Alkohole wie Cyclohexanol, Ketone, wie Aceton oder Acetylaceton, Cycloketone, wie Cyclohexanon, Sulfolen oder Mischungen davon.

Es hat sich überraschend gezeigt, dass durch das oben beschriebene Verfahren Gerüstmaterialien in vergleichsweise hoher Ausbeute und vergleichsweise hohen spezifischen Oberflächen hergestellt werden können. Weiterhin ist ein leichtes Scale-up möglich, da für die Umsetzung kein erhöhter Druck erforderlich ist.

Ein weiterer Gegenstand der vorliegenden Erfindung ist ein poröses metallorganisches Gerüstmaterial erhältlich aus dem erfindungsgemäßen Verfahren.

Es hat sich unter anderem als vorteilhaft herausgestellt, wenn die Umsetzung unter Rühren stattfinden kann, was auch bei einem Scale-up vorteilhaft ist und typischerweise nicht unter Verwendung von Solvo- bzw. Hydrothermalbedingungen durchgeführt wird.

Die Umsetzung in dem erfindungsgemäßen Verfahren findet bei Atmosphärendruck statt. Es ist also kein erhöhter Druck für die Durchführung der Reaktion erforderlich. Insbesondere ist es nicht erforderlich unter erhöhtem Druck zu arbeiten, um zu höheren spezifischen Oberflächen zu gelangen. Insbesondere ist es nicht erforderlich unter Solvothermalbedingungen zu arbeiten. Obwohl die Reaktion bei Atmosphärendruck durchgeführt wird, kann es apparativ bedingt zu leichten Über- oder Unterdrücken während der Umsetzung kommen. Unter dem Begriff "Atmosphärendruck" ist daher im Rahmen der vorliegenden Erfindung ein Druckbereich zu verstehen, der durch einen nach oben und unten um höchstens 250 mbar, vorzugsweise höchstens 200 mbar, abweichenden Atmosphärendruck definiert wird. Der tatsächliche Druck bei der Umsetzung liegt somit in dem oben angegebenen Bereich. Weiterhin bevorzugt ist der tatsächliche Druck gleich dem Atmosphärendruck.

Die Umsetzung erfolgt für die erfindungsgemäße Herstellung eines porösen metallorganischen Gerüstmaterials im Bereich von 90 °C bis 150 °C, besonders bevorzugt im Bereich von 100 °C bis 130 °C und insbesondere bevorzugt im Bereich von 105 °C und 115 °C. Die Temperatur sollte 200 °C, vorzugsweise 180 °C, nicht übersteigen.

Vorzugsweise beträgt die Dauer der Umsetzung 1 bis 72 Stunden, weiter bevorzugt 2 bis 24 Stunden und ganz besonders bevorzugt 3 bis 12 Stunden. Die Umsetzung kann jedoch auch für eine größere Zeitspanne erfolgen.

Bei der eingesetzten Kupferverbindung handelt es sich um eine Kupfer(I)- oder Kupfer(II)-Verbindung. Vorzugsweise handelt es sich um eine Kupfer(II)-Verbindung, insbesondere in Form eines Salzes.

Ein Vorteil der vorliegenden Erfindung liegt darin, dass nicht - wie im Stand der Technik-Kupfer(II)-nitrat in seiner hydratisierten Form eingesetzt werden muss.

Ein Gegenstand der vorliegenden Erfindung ist daher ein Verfahren zur Herstellung eines porösen metallorganischen Gerüstmaterials, wobei die Kupferverbindung eine andere als Kupfer(II)-nitrat ist. Insbesondere ist bevorzugt, wenn nicht eines der Hydrate eingesetzt wird.

Vorzugsweise ist die Kupfer(II)-Verbindung ausgewählt aus der Gruppe bestehend aus Kupfer(II)-formiat, -acetat, -acetylacetonat, -sulfat, -bromid, -chlorid, -carbonat, -und-tartrat. Besonders bevorzugt ist Kupfer(II)-sulfat.

Weiterhin bevorzugt ist es, wenn diese Kupferverbindungen in wasserfreier Form eingesetzt werden. Dies gilt auch allgemein für den Einsatz anderer Kupferverbindungen.

Die Kupferverbindung wird mit mindestens einer mindestens zweizähnigen Verbindung umgesetzt, die koordinativ an das Kupfer binden kann. Die mindestens eine mindestens zweizähnige organische Verbindung leitet sich von einer Polycarbonsäure mit mindestens drei Carboxylgruppen ab und ist eine Tri- oder Tetracarbonsäure. Die mindestens drei Carboxylgruppen sowie weitere funktionelle Gruppen können grundsätzlich an jede geeignete organische Verbindung gebunden sein, solange gewährleistet ist, dass diese funktionellen Gruppen aufweisende organische Verbindungen zur Ausbildung der koordinativen Verbindung und zur Herstellung des Gerüstmaterials befähigt ist.

Bevorzugt leiten sich die organischen Verbindungen, die eine Tri- oder Tetracarbonsäure ist, von einer gesättigten oder ungesättigten aliphatischen Verbindung oder einer aromatischen Verbindung oder einer sowohl aliphatischen als auch aromatischen Verbindung ab.

Die aliphatische Verbindung oder der aliphatische Teil der sowohl aliphatischen als auch aromatischen Verbindung kann linear und/oder verzweigt und/oder cyclisch sein, wobei auch mehrere Cyclen pro Verbindung möglich sind. Weiter bevorzugt enthält die aliphatische Verbindung oder der aliphatische Teil der sowohl aliphatischen als auch aromatischen Verbindung 1 bis 18, weiter bevorzugt 1 bis 14, weiter bevorzugt 1 bis 13, weiter bevorzugt 1 bis 12, weiter bevorzugt 1 bis 11 und insbesondere bevorzugt 1 bis 10 C-Atome wie beispielsweise 1, 2, 3, 4, 5, 6, 7, 8, 9 oder 10 C-Atome. Insbesondere bevorzugt sind hierbei unter anderem Methan, Adamantan, Acetylen, Ethylen oder Butadien.

Die aromatische Verbindung oder der aromatische Teil der sowohl aromatischen als auch aliphatischen Verbindung kann einen oder auch mehrere Kerne wie beispielsweise zwei, drei, vier oder fünf Kerne aufweisen, wobei die Kerne getrennt voneinander und/oder mindestens zwei Kerne in kondensierter Form vorliegen können. Besonders bevorzugt weist die aromatische Verbindung oder der aromatische Teil der sowohl aliphatischen als auch aromatischen Verbindung einen, zwei oder drei Kerne auf, wobei ein oder zwei Kerne besonders bevorzugt sind. Unabhängig voneinander kann weiter jeder Kern der genannten Verbindung mindestens ein Heteroatom wie beispielsweise N, O, S, B, P, Si, bevorzugt N, O und/oder S enthalten. Weiter bevorzugt enthält die aromatische Verbindung oder der aromatische Teil der sowohl aromatischen als auch aliphatischen Verbindung einen oder zwei C₆-Kerne, wobei die zwei entweder getrennt voneinander oder in kondensierter Form vorliegen. Insbesondere sind als aromatische Verbindungen Benzol, Naphthalin und/oder Biphenyl und/oder Bipyridyl und/oder Pyridin zu nennen.

Der Begriff "ableiten" bedeutet im Rahmen der vorliegenden Erfindung, dass die mindestens zweizähnige organische Verbindung im Gerüstmaterial in teilweise depronitonierter oder vollständig deprotinierter Form vorliegen kann. Weiterhin kann die mindestens zweizähnige organische Verbindung weitere Substituenten enthalten, wie beispielsweise -OH,-SH, -NH₂, -OCH₃, -CH₃, -NH(CH₃), -N(CH₃)₂, -CN sowie Halogenide. Darüber hinaus bedeutet der Begriff "ableiten", dass die Carboxylgruppen unabhängig voneinander in Form ihrer Schwefelanaloga vorliegen können. Schwefelanaloga sind die funktionellen Gruppen -C(=O)SH sowie dessen Tautomer und -C(=S)SH.

Die mindestens eine mindestens zweizähnige organische Verbindung ist eine Tri- oder Tetracarbonsäure.

Weiterhin mehr bevorzugt ist die mindestens zweizähnige organische Verbindung ein aliphatischer oder aromatischer azyklischer oder zyklischer Kohlenwasserstoff mit 1-18 Kohlenstoffatomen, der zudem ausschließlich mindestens drei Carboxylgruppen als funktionelle Gruppen aufweist.

Beispielsweise sind im Rahmen der vorliegenden Erfindung

Tricarbonsäuren wie etwa 2-Hydroxy-1,2,3-propantricarbonsäure, 7-Chlor-2,3,8-chinolintricarbonsäure, 1,2,3-, 1,2,4-Benzoltricarbonsäure, 1,2,4-Butantricarbonsäure, 2-Phosphono-1,2,4-butantri-carbonsäure, 1,3,5-Benzoltricarbonsäure, 1-Hydroxy-1,2,3-Propantricarbonsäure, 4,5-Dihydro-4,5-dioxo-1H-pyrrolo[2,3-F]chinolin-2,7,9-tricarbonsäure, 5-Acetyl-3-amino-6-methylbenzol-1,2,4-tricarbonsäure, 3-Amino-5-benzoyl-6-methylbenzol-1,2,4-tricarbonsäure, 1,2,3-Propantricarbonsäure oder Aurintricarbonsäure,
oder Tetracarbonsäuren wie etwa
1,1-Dioxidperylo[1,12-BCD]thiophen-3,4,9,10-tetracarbonsäure, Perylentetracarbon-säuren wie Perylen-3,4,9,10-tetracarbonsäure oder Perylen-1,12-sulfon-3,4,9,10-tetracarbonsäure, Butantetracarbonsäuren wie 1,2,3,4-Butantetracarbonsäure oder Meso-1,2,3,4-Butantetracarbonsäure, Decan-2,4,6,8-tetracarbonsäure, 1,4,7,10,13,16-Hexaoxacyclooctadecan-2,3,11,12-tetracarbonsäure, 1,2,4,5-Benzoltetracarbonsäure, 1,2,11,12-Dodecantetracarbonsäure, 1,2,5,6-Hexan-tetracarbonsäure, 1,2,7,8-Octantetracarbonsäure, 1,4,5,8-Naphthalintetracarbonsäure, 1,2,9,10-Decantetracarbon-säure, Benzophenonietracarbonsäure, 3,3',4,4'-Benzo-phenontetracarbonsäure, Tetrahydrofurantetracarbonsäure oder Cyclopentantetracarbonsäuren wie Cyclopentan-1,2,3,4-tetracarbonsäure zu nennen.

Ganz besonders bevorzugt werden gegebenenfalls mindestens einfach substituierte mono-, di-, tri-, tetra- oder höherkernige aromatische Tri- oder Tetracarbonsäuren eingesetzt, wobei jeder der Kerne mindestens ein Heteroatom enthalten kann, wobei zwei oder mehr Kerne gleiche oder unterschiedliche Heteroatome enthalten können. Beispielsweise bevorzugt werden monokernige Tricarbonsäuren, monokernige Tetracarbonsäuren, dikernige Tricarbonsäuren, dikernige Tetracarbonsäuren, trikernige Tricarbonsäuren, trikernige Tetracarbonsäuren, tetrakernige Tricarbonsäuren und/oder tetrakernige Tetracarbonsäuren. Geeignete Heteroatome sind beispielsweise N, O, S, B, P; bevorzugte Heteroatome sind hierbei N, S und/oder O. Als geeigneter Substituent ist diesbezüglich unter anderem -OH, eine Nitrogruppe, eine Aminogruppe oder eine Alkyl- oder Alkoxygruppe zu nennen.

Insbesondere bevorzugt als mindestens eine mindestens zweizähnige organische Verbindung ist 1,2,3-Benzoltricarbonsäure, 1,2,4-Benzoltricarbonsäure oder 1,3,5-Benzoltricarbonsäure.

Neben diesen mindestens zweizähnigen organischen Verbindungen kann das metallorganische Gerüstmaterials auch einen oder mehrere einzähnige Liganden umfassen.

Das metallorganische Gerüstmaterial kann als Polymer oder als so genanntes "beschränktes" Gerüstmaterial anfallen.

Das nicht-wässrige organische Lösemittel ist C₄₋₁₀-Alkanol, Dimethylsulfoxid (DMSO), N,N-Dimethylformamid (DMF), N,N-Diethylformamid (DEF), N,N-Dimethylacetamid (DMAc), Acetonitril, Toluol, Dioxan, Chlorbenzol, Methylethylketon (MEK), Pyridin, gegebenenfalls halogeniertes C₇₋₂₀₀-Alkan, Sulfolan, Alkylenpolyole, wie Ethylenglykol, Polyalkylenpolyole, wie Polyethylenglykol, Glycerin, Propylencarbonat, N-Methylpyrrolidon (NMP), gamma-Butyrolacton, alicyclische Alkohole wie Cyclohexanol, Ketone, wie Aceton oder Acetylaceton, Cycloketone, wie Cyclohexanon, Sulfolen oder Mischungen davon.

Das nicht-wässrige Lösemittel wird derart gewählt, dass eine Reaktionstemperatur von oberhalb 80 °C unter Normaldruck erreicht werden kann. Sollte die Siedetemperatur eines Lösemittels oder -gemisches nicht ausreichend hoch sein, kann gegebenenfalls das Zumischen eines höhersiedenden Lösemittels die gewünschte Mindesttemperatur ermöglichen. Das Reaktionsgemisch wird bevorzugt unterhalb der Siedetemperatur (unter Rückfluss) gehalten. Dies ist jedoch nicht zwingend erforderlich.

Ein C₄₋₁₀-Alkanol bezeichnet einen Alkylalkohol mit 4 bis 10 C-Atomen. Beispiele hierfür sind n-Butanol, i-Butanol, t-Butanol, Pentanol, Hexanol, Heptanol, Oktanol, Nonanol, Dekanol sowie Gemische davon.

Ein gegebenenfalls halogeniertes C₇₋₂₀₀-Alkan bezeichnet ein Alkan mit 7 bis 200 C-Atomen, wobei 1 oder mehrere bis hin zu allen Wasserstoffatomen durch Halogen, vorzugsweise Chlor oder Fluor, insbesondere Chlor ersetzt sein kann beziehungsweise können. Beispiele hierfür sind Heptan, 1,1,1-Trichlorheptan, Oktan, Nonan, Dekan, Undekan, Dodekan sowie Gemische davon.

Bevorzugte Lösemittel sind Alkylenpolyole, Polyalkylenpolyole, DMF, DEF und NMP. Besonders bevorzugt ist Ethylenglykol.

Der Begriff "nicht-wässrig" bezieht sich vorzugsweise auf ein Lösemittel, das einen Höchstwassergehalt von 10 Gew.-%, mehr bevorzugt 5 Gew.-%, weiterhin mehr bevorzugt 1 Gew.-%, weiterhin bevorzugt 0,1 Gew.-%, besonders bevorzugt 0,01 Gew.-% bezogen auf das Gesamtgewicht des Lösemittels nicht überschreitet.

Vorzugsweise beträgt der gesamte Höchstwassergehalt in der Flüssigphase während der Umsetzung 10 Gew.-%, mehr bevorzugt 5 Gew.-% und weiterhin mehr bevorzugt 1 Gew.-%, insbesondere 0,5 Gew.-%.

Der Begriff "Lösemittel" betrifft reine Lösemittel sowie Gemische von unterschiedlichen Lösemitteln.

Die Entfernung der mindestens zweizähnigen organischen Verbindung (Ligand) aus den Poren des porösen metallorganischen Gerüstmaterials kann durch die Behandlung des gebildeten Gerüstmaterials mit einem nicht-wässrigen Lösemittel erfolgen. Hierbei wird in einer Art "Extraktionsverfahren" der Ligand entfernt und gegebenenfalls im Gerüstmaterial durch ein Lösemittelmolekül ersetzt. Diese schonende Methode ist insbesondere geeignet, wenn es sich bei dem Liganden um eine hochsiedende Verbindung handelt.

Die Behandlung erfolgt vorzugsweise mindestens 30 Minuten und kann typischerweise bis zu 2 Tagen durchgeführt werden. Dies kann bei Raumtemperatur oder erhöhter Temperatur geschehen. Vorzugsweise erfolgt dies unter erhöhter Temperatur, beispielsweise bei mindestens 40 °C, bevorzugt 60 °C. Weiterhin bevorzugt erfolgt die Extraktion bei der Siedetemperatur des eingesetzten Lösemittels statt (unter Rückfluss).

Die Behandlung kann in einem einfachen Kessel durch Aufschlämmen und Rühren des Gerüstmaterials erfolgen. Es können auch Extraktionsapparaturen wie Soxhlet-Apparaturen, insbesondere technische Extraktionsapparaturen, eingesetzt werden.

Als geeignete Lösemittel können die oben genannten verwendet werden. Es können jedoch auch weitere Lösemittel verwendet werden. Beispiele sind C₁₋₆-Alkanol, Dimethylsulfoxid (DMSO), N,N-Dimethylformamid (DMF), N,N-Diethylformamid (DEF), Acetonitril, Toluol, Dioxan, Benzol, Chlorbenzol, Methylethylketon (MEK), Pyridin, Tetrahydrofuran (THF), Essigsäureethylester, gegebenenfalls halogeniertes C₁₋₂₀₀-Alkan, Sulfolan, Glykol, N-Methylpyrrolidon (NMP), gamma-Butyrolacton, alicyclische Alkohole wie Cyclohexanol, Ketone, wie Aceton oder Acetylaceton, Cycloketone, wie Cyclohexanon oder Mischungen davon.

Ein C₁₋₆-Alkanol bezeichnet einen Alkohol mit 1 bis 6 C-Atomen. Beispiele hierfür sind Methanol, Ethanol, n-Propanol, i-Propanol, n-Butanol, i-Butanol, t-Butanol, Pentanol, Hexanol sowie Gemische davon.

Ein gegebenenfalls halogenisiertes C₁₋₂₀₀-Alkan bezeichnet ein Alkan mit 1 bis 200 C-Atomen, wobei ein oder mehrere bis hin zu allen Wasserstoffatomen durch Halogen, vorzugsweise Chlor oder Fluor, insbesondere Chlor, ersetzt sein kann bzw. können. Beispiele hierfür sind Chloroform, Dichlormethan, Tetrachlormethan, Dichlorethan, Hexan, Heptan, Oktan sowie Gemische davon.

Soll alternativ oder darüber hinaus das Lösemittel, welches bei der Umsetzung verwendet wurde aus den Poren entfernt werden, ist bevorzugt dass nach der Umsetzung das gebildete metallorganische Gerüstmaterial mit einem weiteren organischen Lösemittel, welches einen niedrigeren Siedepunkt als das bei der Umsetzung verwendete nicht-wässrige organische Lösemittel aufweist, behandelt wird, um gegebenenfalls in den Poren des metallorganischen Gerüstmaterials vorhandene mindestens zweizähnige Verbindung oder nicht-wässriges organisches Lösemittel aus der Umsetzung zu entfernen.

Bevorzugt sind Lösemittel oder Gemische davon, die einen Siedepunkt bei Normaldruck unter 80 °C aufweisen.

Bevorzugt sind insbesondere Methanol, Ethanol, Propanol, Aceton, MEK und Mischungen davon.

Ein ganz besonders bevorzugtes Extraktionslösemittel ist Methanol.

Das verwendete Lösemittel zur Extraktion kann gleich oder verschieden zu demjenigen für die Umsetzung der mindestens einen Metallverbindung mit der mindestens einen mindestens zweizähnigen organischen Verbindung sein. Insbesondere ist es bei der "Extraktion" nicht unbedingt erforderlich aber bevorzugt, dass das Lösemittel wasserfrei ist.

Der Extraktion kann ein Trocknungsschritt vor- und/oder nachgeschaltet sein. Hierbei sollte eine Temperatur von 250 °C nicht überschritten werden.

Weiterhin ist bevorzugt, wenn während der Umsetzung der Flüssigphase Wasser entzogen wird. Die Flüssigphase besteht normalerweise aus dem nicht-wässrigen Lösemittel sowie aus bei der Umsetzung gebildetem Wasser.

Das Entfernen des Wassers aus dem Reaktionsgemisch kann insbesondere destillativ, durch Strippen oder durch Adsorptionsmittel erfolgen. Beim Strippen (oder Austreiben genannt) werden aus einer Flüssigphase Inhaltsstoffe dieser Flüssigphase durch das Durchleiten von Gasen aus der Flüssigphase entfernt und in eine Gasphase überführt. Geeignete Adsorptionsmittel sind beispielsweise Aluminiumoxid, Kieselgel oder ein Molsieb, insbesondere ein 3 Å- oder 4 Å-Molsieb.

Die metallorganischen Gerüstmaterialien gemäß der vorliegenden Erfindung enthalten Poren, insbesondere Mikro- und/oder Mesoporen. Mikroporen sind definiert als solche mit einem Durchmesser von 2 nm oder kleiner und Mesoporen sind definiert durch einen Durchmesser im Bereich von 2 bis 50 nm, jeweils entsprechend nach der Definition, wie sie Pure Applied Chem. 45, Seite 71, insbesondere auf Seite 79 (1976) angegeben ist. Die Anwesenheit von Mikro- und/oder Mesoporen kann mit Hilfe von Sorptionsmessungen überprüft werden, wobei diese Messungen die Aufnahmekapazität der MOF für Stickstoff bei 77 Kelvin gemäß DIN 66131 und/oder DIN 66134 bestimmt.

Wie bereits oben ausgeführt weisen die erfindungsgemäßen metallorganischen Gerüstmaterialien eine hohe spezifische Oberfläche auf. Die spezifische Oberfläche der erfindungsgemäßen metallorganischen Gerüstmaterialien in Pulverform beträgt vorzugsweise mehr als 1500 m²/g nach Langmuir (N₂) gemäß DIN 66135 (DIN 66131, 66134). Mehr bevorzugt beträgt die spezifische Oberfläche mehr als 1700 m²/g, weiterhin bevorzugt mehr als 1800 m²/g, weiterhin bevorzugt mehr als 1850 m²/g und insbesondere bevorzugt mehr als 1900 m²/g.

Gerüstmaterialien, die als Formkörper vorliegen, können eine niedrigere spezifische Oberfläche besitzen.

Bevorzugt beträgt weiterhin für das erfindungsgemäße poröse metallorganische Gerüstmaterial das Porenvolumen (nach Bestimmung mit N₂) wenigstens 0,5 ml/g, mehr bevorzugt wenigstens 0,6 ml/g. Der bevorzugte mittlere Porenradius (nach Bestimmung mit N₂) liegt vorzugsweise zwischen 0,8 und 10 mm, mehr bevorzugt zwischen 10 und 30 mm.

Das metallorganische Gerüstmaterial kann pulverförmig beziehungsweise als Agglomerat vorliegen. Das Gerüstmaterial kann als solches verwendet werden oder es wird in einen Formkörper umgewandelt. Demgemäß ist ein weiterer Aspekt der vorliegenden Erfindung ein Formkörper enthaltend ein erfindungsgemäßes Gerüstmaterial.

Bevorzugte Verfahren zur Herstellung von Formkörpern sind hierbei die Verstrangung oder Tablettierung. Bei der Formkörperherstellung kann das Gerüstmaterial weitere Materialien, wie beispielsweise Binder, Gleitmittel oder andere Additive aufweisen, welche während der Herstellung hinzugesetzt werden. Ebenso ist es denkbar, dass das Gerüstmaterial weitere Bestandteile aufweist, wie zum Beispiel Absorbentien wie Aktivkohle oder dergleichen.

Hinsichtlich der möglichen Geometrien der Formkörper existieren im Wesentlichen keine Beschränkungen. Beispielsweise sind unter anderem Pellets wie beispielsweise scheibenförmige Pellets, Pillen, Kugeln, Granulat, Extrudate wie beispielsweise Stränge, Waben, Gitter oder Hohlkörper zu nennen.

Zur Herstellung dieser Formkörper sind grundsätzlich sämtliche geeigneten Verfahren möglich. Es sind insbesondere folgende Verfahrensführungen bevorzugt:
- Kneten/Kollern des Gerüstmaterials allein oder zusammen mit mindestens einem Bindemittel und/oder mindestens einem Anteigungsmittel und/oder mindestens
   einer Templatverbindung unter Erhalt eines Gemisches; Verformen des erhaltenen Gemisches mittels mindestens einer geeigneten Methode wie beispielsweise Extrudieren; Optional Waschen und/oder Trocknen und/oder Calcinieren des Extrudates; Optional Konfektionieren.
- Tablettieren zusammen mit mindestens einem Bindemittel und/oder anderem Hilfsstoff.
- Aufbringen des Gerüstmaterials auf mindestens ein gegebenenfalls poröses Trägermaterial. Das erhaltene Material kann dann gemäß der vorstehend beschriebenen Methode zu einem Formkörper weiterverarbeitet werden.
- Aufbringen des Gerüstmaterials auf mindestens ein gegebenenfalls poröses Substrat.

Kneten/Kollern und Verformen kann gemäß eines jeden geeigneten Verfahrens erfolgen, wie beispielsweise in Ullmanns Enzyklopädie der Technischen Chemie, 4. Auflage, Band 2, S. 313 ff. (1972) beschrieben.

Beispielsweise kann das Kneten/Kollern und/oder Verformen mittels einer Kolbenpresse, Walzenpresse in Anwesenheit oder Abwesenheit mindestens eines Bindermaterials, Compoundieren, Pelletieren, Tablettieren, Extrudieren, Co-Extrudieren, Verschäumen, Verspinnen, Beschichten, Granulieren, bevorzugt Sprühgranulieren, Versprühen, Sprühtrocknen oder einer Kombination aus zwei oder mehr dieser Methoden erfolgen.

Ganz besonders bevorzugt werden Pellets und/oder Tabletten hergestellt.

Das Kneten und/oder Verformen kann bei erhöhten Temperaturen wie beispielsweise im Bereich von Raumtemperatur bis 300 °C und/oder bei erhöhtem Druck wie beispielsweise im Bereich von Normaldruck bis hin zu einigen hundert bar und/oder in einer Schutzgasatmosphäre wie beispielsweise in Anwesenheit mindestens eines Edelgases, Stickstoff oder einem Gemisch aus zwei oder mehr davon erfolgen.

Das Kneten und/oder Verformen wird gemäß einer weiteren Ausführungsform unter Zugabe mindestens eines Bindemittels durchgeführt, wobei als Bindemittel grundsätzlich jede chemische Verbindung eingesetzt werden kann, die die zum Kneten und/oder Verformen gewünschte Viskosität der zu verknetenden und/oder verformenden Masse gewährleistet. Demgemäß können Bindemittel im Sinne der vorliegenden Erfindung sowohl Viskositätserhöhende als auch Viskositätserniedrigende Verbindungen sein.

Als unter anderem bevorzugte Bindemittel sind beispielsweise Aluminiumoxid oder Aluminiumoxid enthaltende Binder, wie sie beispielsweise in der WO 94/29408 beschrieben sind, Siliciumdioxid, wie es beispielsweise in der EP 0 592 050 A1 beschrieben ist, Mischungen ais Siliciumdioxid und Aluminiumoxid, wie sie beispielsweise in der WO 94/13584 beschrieben sind, Tonminerale, wie sie beispielsweise in der JP 03-037156 A beschrieben sind, beispielsweise Montmorillonit, Kaolin, Bentonit, Halloysit, Dickit, Nacrit und Anauxit, Alkoxysilane, wie sie beispielsweise in der EP 0 102 544 B1 beschrieben sind, beispielsweise Tetraalkoxysilane wie beispielsweise Tetramethoxysilan, Tetraethoxysilan, Tetrapropoxysilan, Tetrabutoxysilan, oder beispielsweise Trialkoxysilane wie beispielsweise Trimethoxysilan, Triethoxysilan, Tripropoxysilan, Tributoxysilan, Alkoxytitanate, beispielsweise Tetraalkoxytitanate wie beispielsweise Tetramethoxytitanat, Tetraethoxytitanat, Tetrapropoxytitanat, Tetrabutoxytitanat, oder beispielsweise Trialkoxytitanate wie beispielsweise Trimethoxytitanat, Triethoxytitanat, Tripropoxytitanat, Tributoxytitanat, Alkoxyzirkonate, beispielsweise Tetraalkoxyzirkonate wie beispielsweise Tetramethoxyzirkonat, Tetraethoxyzirkonat, Tetrapropoxyzirkonat, Tetrabutoxyzirkonat, oder beispielsweise Trialkoxyzirkonate wie beispielsweise Trimethoxyzirkonat, Triethoxyzirkonat, Tripropoxyzirkonat, Tributoxyzirkonat, Silikasole, amphiphile Substanzen und/oder Graphite zu nennen.

Als viskositätssteigernde Verbindung kann beispielsweise auch, gegebenenfalls zusätzlich zu den oben genannten Verbindungen, eine organische Verbindung und/oder ein hydrophiles Polymer wie beispielsweise Cellulose oder ein Cellulosederivat wie beispielsweise Methylcellulose und/oder ein Polyacrylat und/oder ein Polymethacrylat und/oder ein Polyvinylalkohol und/oder ein Polyvinylpyrrolidon und/oder ein Polyisobuten und/oder ein Polytetrahydrofuran und/oder ein Polyethylenoxid eingesetzt werden.

Als Anteigungsmittel kann unter anderem bevorzugt Wasser oder mindestens ein Alkohol wie beispielsweise ein Monoalkohol mit 1 bis 4 C-Atomen wie beispielsweise Methanol, Ethanol, n-Propanol, iso-Propanol, 1-Butanol, 2-Butanol, 2-Methyl-1-propanol oder 2-Methyl-2-propanol oder ein Gemisch aus Wasser und mindestens einem der genannten Alkohole oder ein mehrwertiger Alkohol wie beispielsweise ein Glykol, bevorzugt ein wassermischbarer mehrwertiger Alkohol, allein oder als Gemisch mit Wasser und/oder mindestens einem der genannten einwertigen Alkohole eingesetzt werden.

Weitere Additive, die zum Kneten und/oder Verformen eingesetzt werden können, sind unter anderem Amine oder Aminderivate wie beispielsweise Tetraalkylammonium-Verbindungen oder Aminoalkohole und Carbonat enthaltende Verbindungen wie etwa Calciumcarbonat. Solche weiteren Additive sind etwa in der EP 0 389 041 A1, der EP 0 200 260 A1 oder der WO 95/19222 beschrieben.

Die Reihenfolge der Additive wie Templatverbindung, Binder, Anteigungsmittel, viskositätssteigernde Substanz beim Verformen und Kneten ist grundsätzlich nicht kritisch.

Gemäß einer weiteren bevorzugten Ausführungsform wird der gemäß Kneten und/oder Verformen erhaltene Formkörper mindestens einer Trocknung unterzogen, die im Allgemeinen bei einer Temperatur im Bereich von 20 bis 400 °C, bevorzugt im Bereich von 30 bis 300 °C und besonders bevorzugt im Bereich von 80 bis 200 °C durchgeführt wird. Ebenso ist es möglich, im Vakuum oder unter Schutzgasatmosphäre oder durch Sprühtrocknung zu trocknen.

Gemäß einer besonders bevorzugten Ausführungsform wird im Rahmen dieses Trocknungsvorgangs mindestens eine der als Additive zugesetzten Verbindungen zumindest teilweise aus dem Formkörper entfernt.

Ein weiterer Gegenstand der vorliegenden Erfindung ist die Verwendung des erfindungsgemäßen porösen metallorganischen Gerüstmaterials zur Aufnahme mindestens eines Stoffes zu dessen Speicherung, Abtrennung, kontrollierten Abgabe oder chemischen Umsetzung sowie als Trägermaterial.

Bei dem mindestens einen Stoff kann es sich um ein Gas oder eine Flüssigkeit handeln. Vorzugsweise handelt es sich bei dem Stoff um ein Gas.

Im Rahmen der vorliegenden Erfindung werden vereinfachend die Begriffe "Gas" und "Flüssigkeit" verwendet, wobei hier jedoch ebenso Gasgemische sowie Flüssigkeitsgemische beziehungsweise flüssige Lösungen unter dem Begriff "Gas" beziehungsweise "Flüssigkeit" zu verstehen sind.

Bevorzugte Gase sind Wasserstoff, Kohlenwasserstoffe, insbesondere Methan, Ethan, Ethen, Acetylen, Propan, n-Butan sowie i-Butan, Kohlenmonoxid, Kohlendioxid, Stickoxide, Sauerstoff, Schwefeloxide, Halogene, halogenierte Kohlenwasserstoffe, NF₃, SF₆, Ammoniak, Borane, Phosphane, Schwefelwasserstoff, Amine, Formaldehyd, Edelgase, insbesondere Helium, Neon, Argon, Krypton sowie Xenon.

Insbesondere bevorzugt ist die Verwendung zur Trennung von Gasgemischen, beispielsweise durch Druck- oder Temperaturwechseladsorption.

Bei dem mindestens einen Stoff kann es sich jedoch auch um eine Flüssigkeit handeln. Beispiele für eine solche Flüssigkeit sind Desinfektionsmittel, anorganische oder organische Lösemittel, Treibstoffe - insbesondere Benzin oder Diesel -, Hydraulik-, Kühler-, Bremsflüssigkeit oder ein Öl, insbesondere Maschinenöl. Weiterhin kann es sich bei der Flüssigkeit um halogenierte aliphatische oder aromatische, cyclische oder acyclische Kohlenwasserstoffe oder Mischungen davon handeln. Insbesondere kann die Flüssigkeit Aceton, Acetonitril, Anilin, Anisol, Benzol, Benzonitril, Brombenzol, Butanol, tert.-Butanol, Chinolin, Chlorbenzol, Chloroform, Cyclohexan, Diethylenglykol, Diethylether, Dimethylacetamid, Dimethylformamid, Dimethylsulfoxid, Dioxan, Eisessig, Essigsäureanhydrid, Essigsäureethylester, Ethanol, Ethylencarbonat, Ethylendichlorid, Ethylenglykol, Ethylenglykoldimethylether, Formamid, Hexan, Isopropanol, Methanol, Methoxypropanol, 3-Methyl-1-butanol, Methylenchlorid, Methylethylketon, N-Methylformamid, N-Methylpyrrolidon, Nitrobenzol, Nitromethan, Piperidin, Propanol, Propylencarbonat, Pyrridin, Schwefelkohlenstoff, Sulfolan, Tetrachlorethen, Tetrachlorkohlenstoff, Tetrahydrofuran, Toluol, 1,1,1-Trichlorethan, Trichlorethylen, Triethylamin, Triethylenglykol, Triglyme, Wasser oder Mischungen hiervon handeln.

Weiterhin kann der mindestens eine Stoff ein Geruchsstoff sein.

Vorzugsweise handelt es sich bei dem Geruchsstoff um eine flüchtige organische oder anorganische Verbindung, die mindestens eines der Elemente Stickstoff, Phosphor, Sauerstoff, Schwefel, Fluor, Chlor, Brom oder lod enthält oder ein ungesättigter oder aromatischer Kohlenwasserstoff oder ein gesättigter oder ungesättigter Aldehyd oder ein Keton ist. Mehr bevorzugte Elemente sind Stickstoff, Sauerstoff, Phosphor, Schwefel, Chlor, Brom; insbesondere bevorzugt sind Stickstoff, Sauerstoff, Phosphor und Schwefel.

Insbesondere handelt es sich bei dem Geruchsstoff um Ammoniak, Schwefelwasserstoff, Schwefeloxide, Stickoxide, Ozon, cyclische oder acyclische Amine, Thiole, Thioether sowie Aldehyde, Ketone, Ester, Ether, Säuren oder Alkohole. Besonders bevorzugt sind Ammoniak, Schwefelwasserstoff, organische Säuren (bevorzugt Essigsäure, Propionsäure, Buttersäure, Isobuttersäure, Valeriansäure, Isovaleriansäure, Capronsäure, Heptylsäure, Laurinsäure, Pelargonsäure) sowie cyclische oder acyclische Kohlenwasserstoffe, die Stickstoff oder Schwefel enthalten sowie gesättigte oder ungesättigte Aldehyde, wie Hexanal, Heptanal, Oktanal, Nonanal, Decanal, Octenal oder Nonenal und insbesondere flüchtige Aldehyde wie Butyraldehyd, Propionaldehyd, Acetaldehyd und Formaldehyd und weiterhin Treibstoffe wie Benzin, Diesel (Inhaltsstoffe).

Bei den Geruchsstoffen kann es sich auch um Riechstoffe, die beispielsweise zur Herstellung von Parfümen verwendet werden, handeln. Beispielhaft seien als Riechstoffe oder Öle, die solche Riechstoffe freisetzen zu nennen: ätherische Öle, Basilikumöl, Geranienöl, Minzöl, Canangabaumöl, Kardamomöl, Lavendelöl, Pfefferminzöl, Muskatöl, Kamillenöl, Eukalyptusöl, Rosmarinöl, Zitronenöl, Limettenöl, Orangenöl, Bergamottenöl, Muskatellersalbeiöl, Korianderöl, Zypressenöl, 1,1-Dimethoxy-2-pherylethan, 2,4-Dimethyl-4-phenyltetrahydrofuran, Dimethyltetrahydrobenzaldehyd, 2,6-Dimethyl-7-octen-2-ol, 1,2-Diethoxy-3,7-dimethyl-2,6-octadien, Phenylacetaldehyd, Rosenoxid, Ethyl-2-methylpentanoat, 1-(2,6,6-Trimethyl-1,3-cyclohexadien-1-yl)-2-buten-1-on, Ethylvanillin, 2,6-Dimethyl-2-octenol, 3,7-Dimethyl-2-octenol, tert-Butylcyclohexylacetat, Anisylacetate, Allylcyclohexyloxyacetat, Ethyllinalool, Eugenol, Cumarin, Ethylacetacetat, 4-Phenyl-2,4,6-trimethyl-1,3-dioxan, 4-Methylen-3,5,6,6-tetramethyl-2-heptanon, Ethyltetrahydrosafranat, Geranylnitril, cis-3-Hexen-1-ol, cis-3-Hexenylacetat, cis-3-Hexenylmethylcarbonate, 2,6-Dimethyl-5-hepten-1-al, 4-(Tricyclo[5.2.1.0]decylidene)-8-butanal, 5-(2,2,3-Trimethyl-3-cyclopentenyl)-3-methylpentan-2-ol, p-tert-Butyl- alpha-methylhydrozimtaldehyd, Ethyl[5.2.1.0]tricyclodecancarboxylat, Geraniol, Citronellol, Citral, Linalool, Linalylacetat, Jonone, Phenylethanol oder Mischungen hiervon.

Im Rahmen der vorliegenden Erfindung weist ein flüchtiger Geruchsstoff vorzugsweise einen Siedepunkt oder Siedepunktsbereich von weniger als 300 °C auf. Mehr bevorzugt ist der Geruchsstoff eine leicht flüchtige Verbindung oder Gemisch. Insbesondere bevorzugt weist der Geruchsstoff einen Siedepunkt oder Siedebereich von weniger als 250 °C, mehr bevorzugt weniger als 230 °C, insbesondere bevorzugt weniger als 200 °C auf.

Bevorzugt sind ebenfalls Geruchsstoffe, die eine hohe Flüchtigkeit aufweisen. Als Maß für die Flüchtigkeit kann der Dampfdruck herangezogen werden. Im Rahmen der vorliegenden Erfindung weist ein flüchtiger Geruchsstoff vorzugsweise einen Dampfdruck von mehr als 0,001 kPa (20°C) auf. Mehr bevorzugt ist der Geruchsstoff eine leicht flüchtige Verbindung oder Gemisch. Insbesondere bevorzugt weist der Geruchsstoff einen Dampfdruck von mehr als 0,01 kPa (20°C) auf, mehr bevorzugt einen Dampfdruck von mehr als 0,05 kPa (20°C) auf. Besonders bevorzugt weisen die Geruchsstoffe einen Dampfdruck von mehr als 0,1 kPa (20°C) auf.

### Beispiele

### Beispiel 1: Herstellung eines Cu-1,3,5-BTC-MOFs in Ethylenglykol

12,2 g 1,3,5-BTC und 13,9 g wasserfreies Kupfersulfat werden in 275 g Ethylenglykol suspendiert und unter Rühren 8 h bei 110°C gehalten. Der blaue Niederschlag wird abfiltriert, und mit 5 x 120 ml Methanol gewaschen. Nach der Trocknung für 24 h bei 75°C im Vakuum (0,2 mbar) werden 6,7 g Produkt erhalten.

Vor der Oberflächenbestimmung wurde die Probe zusätzlich noch bei 110°C evakuiert. Die N₂-Oberfläche liegt lediglich bei 2031 m²/g (Langmuir).

### Beispiel 2: Herstellung eines Cu-1,3,5-BTC-MOFs im Pilotmaßstab (Scale-up)

27,8 kg wasserfreies CuSO₄ werden zusammen mit 12,84 kg 1,3,5-Benzoltricarbonsäure in 330 kg Ethylenglykol suspendiert und mit N₂ abgedeckt. Der Kessel wird auf 110°C gebracht und die Synthesemischung 12 h lang unter Rühren auf dieser Temperatur gehalten. Die Lösung wird auf 50°C abgekühlt und unter N₂-Abdeckung mit einer Drucknutsche filtriert. Der Filterkuchen wird mit 4 x 50 l Methanol gewaschen und 96 h mit Stickstoff trockengeblasen.

Es wurden zwei Batches hergestellt. In dem ersten Ansatz wurden 17 kg in dem zweiten 14,5 kg Material enthalten. Vor der Oberflächenbestimmung wurde die Probe jeweils 2 h bei 110°C evakuiert. Die N₂-Oberfläche liegt bei 2096 bzw. 2073 m²/g (Langmuir). Das XRD von Batch 2 ist in Abb. 1 dargestellt. Das Porenvolumen (bestimmt mit N₂ für Fraktion < 5912 Å) wird zu 0,65 ml/g bestimmt. Der durchschnittliche Porendurchmesser liegt bei 20 Å.

### Beispiel 3: Herstellung eines Cu-1,3,5-BTC-MOFs

244,2 kg CuSO₄ Pentahydrat werden zusammen mit 73,8 kg 1,3,5-Benzoltricarbonsäure in 2200 kg Ethylenglykol suspendiert und mit N₂ abgedeckt. Der Kessel wird auf 110°C gebracht und die Synthesemischung 15 h lang unter Rühren auf dieser Temperatur gehalten. Die Lösung wird bei 110°C unter N₂-Abdeckung mit einer Drucknutsche filtriert. Der Filterkuchen wird mit 2 x 200 l Methanol und 2x 240l Methanol unter Rühren gewaschen. Anschließend wird das Produkt 8h bei 140°C im Vakuum getrocknet.

Die Ausbeute beträgt 46,8 kg. Die N₂-Oberfläche liegt bei 2042 m²/g (Langmuir).

### Beispiel 4: Herstellung eines Cu-1,3,5-BTC-MOFs

150 kg wasserfreies CuSO₄ werden zusammen mit 71 kg 1,3,5-Benzoltricarbonsäure in 2200 kg Ethylenglykol suspendiert und mit N₂ abgedeckt. Der Kessel wird auf 110°C gebracht und die Synthesemischung 15 h lang unter Rühren auf dieser Temperatur gehalten. Die Lösung wird bei 110°C unter N₂-Abdeckung mit einer Drucknutsche filtriert. Der Filterkuchen wird mit 2 x 200 l Methanol und 3x 240l Methanol unter Rühren gewaschen. Anschließend wird das Produkt 10h bei 140°C im Vakuum getrocknet.

Die Ausbeute beträgt 61,1 kg. Die N₂-Oberfläche liegt bei 2064 m²/g (Langmuir).

Vergleichsbeispiel 5: Solvothermale Herstellung eines Cu-1,3,5-BTC-MOFs

14,73 g 1,3,5-BTC in 75 ml Ethylenglykol und 32,6 Cu(NO₃)₂*2,5H₂O in 75 ml H₂O werden zusammen in einem Teflon-Liner 18 h bei 110°C gehalten. Beim Öffnen entweichen nitrose Gase. Der Niederschlag wird abfiltriert, mit Wasser gewaschen und 16 h bei 100°C im Vakuum getrocknet.

Vor der Oberflächenbestimmung wurde die Probe zusätzlich noch bei 80°C evakuiert. Die N₂-Oberfläche liegt lediglich bei 793 m²/g (Langmuir).

### Vergleichsbeispiel 6: Drucklose Herstellung eines Cu-1,3,5 in einem leicht-siedenden Lösemittelgemisch

24,4 g 1,3,5-BTC und 54,3 g Cu(NO₃)₂*2,5H₂O werden in 125 g Wasser und 98,5 Ethanol suspendiert und unter Rühren 24 h drucklos bei 84°C unter Rückfluss gehalten. Der blaue Niederschlag wird abfiltriert, und mit 5 x 400 ml Wasser gewaschen. Nach der Trocknung für 16 h bei 110°C werden 16,24 g Produkt erhalten.

Vor der Oberflächenbestimmung wurde die Probe zusätzlich noch bei 110°C evakuiert. Die N₂-Oberfläche liegt lediglich bei 640 m²/g (Langmuir).

### Beispiel 7: Herstellung von Cu-1,3,5-BTC, Waschen mit Aceton

Ein Cu-1,3,5-BTC-MOF wird wie in Beispiel 1 synthetisiert, jedoch wird die Hälfte des Filterkuchens mit 5x 100 ml Aceton gewaschen.

Vor der Oberflächenbestimmung wurden die Proben zusätzlich noch bei 110°C evakuiert. Die N₂-Oberfläche der Aceton-gewaschenen Hälfte allerdings nur bei 1541 m²/g (Langmuir), die der MeOH-gewaschenen Fraktion bei 1940 m²/g.

## Patentansprüche

1. Verfahren zur Herstellung eines porösen metallorganischen Gerüstmaterials den Schritt enthaltend
Umsetzung eines Reaktionsgemisches in der Flüssigphase aus mindestens einer Kupferverbindung mit mindestens einer mindestens zweizähnigen organischen Verbindung, die koordinativ an das Kupfer binden kann, in Gegenwart eines nicht-wässrigen Lösemittels, wobei die mindestens eine mindestens zweizähnige organische Verbindung eine Tri- oder Tetracarbonsäure ist, die Umsetzung unter Atmosphärendruck in einem Bereich von 90°C bis 150 °C erfolgt, die Kupferverbindung eine andere als Kupfer(II)-nitrat ist und wobei das nicht-wässrige Lösemittel ausgewählt ist aus der Gruppe bestehend aus C₄₋₁₀-Alkanol, Dimethylsulfoxid (DMSO), N,N-Dimethylformamid (DMF), N,N-Diethylformamid (DEF), N,N-Dimethylacetamid (DMAc), Acetonitril, Toluol, Dioxan, Chlorbenzol, Methylethylketon (MEK), Pyridin, gegebenenfalls halogeniertes C₇₋₂₀₀-Alkan, Sulfolan, Alkylenpolyole, wie Ethylenglykol, Polyalkylenpolyole, wie Polyethylenglykol, Glycerin, N-Methylpyrrolidon (NMP), gamma-Butyrolacton, alicyclische Alkohole wie Cyclohexanol, Ketone, wie Aceton oder Acetylaceton, Cycloketone, wie Cyclohexanon, Sulfolen oder Mischungen davon.

2. Verfahren nach Anspruch 1, **dadurch gekennzeichnet, dass** die Umsetzung unter Rühren erfolgt.

3. Verfahren nach Anspruch 1 oder 2, **dadurch gekennzeichnet, dass** die Kupferverbindung ausgewählt ist aus der Gruppe der Kupfer(II)-Verbindungen bestehend aus Kupfer(II)-formiat, -acetat, -acetylacetonat, -sulfat, -bromid, -chlorid, -carbonat, und - tartrat, insbesondere in wasserfreier Form.

4. Verfahren nach einem der Ansprüche 1 bis 3, **dadurch gekennzeichnet, dass** die mindestens eine mindestens zweizähnige organische Verbindung ausgewählt ist aus der Gruppe bestehend aus 1,2,3-Benzoltricarbonsäure, 1,2,4-Benzoltricarbonsäure und 1,3,5-Benzoltricarbonsäure.

5. Verfahren nach einem der Ansprüche 1 bis 4, **dadurch gekennzeichnet, dass** das nicht-wässrige Lösungsmittel ausgewählt ist aus der Gruppe bestehend aus Alkylenpolyolen, Polyalkylenpolyolen, DMF, DEF und NMP.

6. Verfahren nach einem der Ansprüche 1 bis 5, **dadurch gekennzeichnet, dass** das nicht-wässrige Lösungsmittel Ethylenglykol ist.

7. Verfahren nach einem der Ansprüche 1 bis 6, **dadurch gekennzeichnet, dass** die Flüssigphase während der Umsetzung einen Höchstwassergehalt von 10-Gew.% aufweist.

8. Verfahren nach einem der Ansprüche 1 bis 6, **dadurch gekennzeichnet, dass** der Flüssigphase während der Umsetzung Wasser entzogen wird.

9. Verfahren nach einem der Ansprüche 1 bis 8, **dadurch gekennzeichnet, dass** nach der Umsetzung das gebildete metallorganische Gerüstmaterial mit einem weiteren organischen Lösemittel, welches einen niedrigeren Siedepunkt als das bei der Umsetzung verwendete nicht-wässrige organische Lösemittel aufweist, behandelt wird, um gegebenenfalls in den Poren des metallorganischen Gerüstmaterials vorhandene mindestens zweizähnige Verbindung oder nicht-wässriges organisches Lösemittel aus der Umsetzung zu entfernen.

10. Poröses metallorganisches Gerüstmaterial erhältlich aus einem Verfahren nach einem der Ansprüche 1 bis 9.

11. Gerüstmaterial nach Anspruch 10, **dadurch gekennzeichnet, dass** dieses in Pulverform eine spezifische Oberfläche nach Langmuir von mehr als 1500 m²/g aufweist.

12. Verwendung eines Gerüstmaterials nach Anspruch 10 oder 11 zur Aufnahme mindestens eines Stoffes zu dessen Speicherung, Abtrennung, kontrollierten Abgabe oder chemischen Umsetzung sowie als Trägermaterial.

## Claims

1. A method for producing a porous metal-organic framework material comprising the step
reacting a reaction mixture in the liquid phase of at least one copper compound having at least one at least bidentate, organic compound which can bind by coordination to the copper in the presence of a nonaqueous solvent, the at least one at least bidentate, organic compound being a tricarboxylic or tetracarboxylic acid, the reaction proceeding at atmospheric pressure in a range from 90°C to 150°C, the copper compound being different from copper(II) nitrate and the nonaqueous solvent being selected from the group consisting of C₄₋₁₀-alkanol, dimethyl sulfoxide (DMSO), N,N-dimethylformamide (DMF), N,N-diethylformamide (DEF), N,N-dimethylacetamide (DMAc), acetonitrile, toluene, dioxane, chlorobenzene, methyl ethyl ketone (MEK), pyridine, optionally halogenated C₇₋₂₀₀-alkane, sulfolane, alkylene polyols such as ethylene glycol, polyalkylene polyols such as polyethylene glycol, glycerol, N-methylpyrrolidone (NMP), gamma-butyrolactone, alicyclic alcohols such as cyclohexanol, ketones such as acetone or acetylacetone, cycloketones such as cyclohexanone, sulfolene or mixtures thereof.

2. The method according to claim 1, wherein the reaction proceeds with stirring.

3. The method according to claim 1 or 2, wherein the copper compound is selected from the group of copper(II) compounds consisting of copper(II) formate, acetate, acetylacetonate, sulfate, bromide, chloride, carbonate and tartrate, in particular in anhydrous form.

4. The method according to one of claims 1 to 3, wherein the at least one at least bidentate, organic compound is selected from the group consisting of 1,2,3-benzenetricarboxylic acid, 1,2,4-benzenetricarboxylic acid and 1,3,5-benzenetricarboxylic acid.

5. The method according to one of claims 1 to 4, wherein the nonaqueous solvent is selected from the group consisting of alkylene polyols, polyalkylene polyols, DMF, DEF and NMP.

6. The method according to one of claims 1 to 5, wherein the nonaqueous solvent is ethylene glycol.

7. The method according to one of claims 1 to 6, wherein the liquid phase during the reaction has a maximum water content of 10% by weight.

8. The method according to one of claims 1 to 6, wherein water is taken off from the liquid phase during the reaction.

9. The method according to one of claims 1 to 8 wherein, after the reaction, the metal-organic framework material formed is treated with a further organic solvent which has a lower boiling point than the nonaqueous organic solvent used in the reaction in order to remove from the reaction any at least bidentate compound or nonaqueous organic solvent present in the pores of the metal-organic framework material.

10. A porous metal-organic framework material obtainable from a method according to one of claims 1 to 9.

11. The framework material according to claim 10, wherein in powder form it has a specific surface area according to Langmuir of greater than 1500 m²/g.

12. The use of a framework material according to claim 10 or 11 for taking up at least one substance for its storage, separation, controlled release or chemical reaction, and also as support material.

## Revendications

1. Procédé de fabrication d'un matériau de squelette métallo-organique poreux contenant les étapes suivantes :
la réaction d'un mélange réactionnel en phase liquide constitué d'au moins un composé de cuivre avec au moins un composé organique au moins bidentate, qui peut se lier coordinativement au cuivre, en présence d'un solvant non aqueux, ledit au moins un composé organique au moins bidentate étant un acide tri- ou tétracarboxylique, la réaction ayant lieu sous pression atmosphérique dans une plage allant de 90 °C à 150 °C, le composé de cuivre étant un autre que le nitrate de cuivre (II) et le solvant non aqueux étant choisi dans le groupe constitué par un alcanol en C₄₋₁₀, le diméthylsulfoxyde (DMSO), le N,N-diméthylformamide (DMF), le N,N-diéthylformamide (DEF), le N,N-diméthylacétamide (DMAc), l'acétonitrile, le toluène, le dioxane, le chlorobenzène, la méthyléthylcétone (MEK), la pyridine, un alcane en C₇₋₂₀₀ éventuellement halogéné, le sulfolane, les alkylène polyols, tels que l'éthylène glycol, les polyalkylène polyols, tels que le polyéthylène glycol, la glycérine, la N-méthylpyrrolidone (NMP), la gamma-butyrolactone, les alcools alicycliques tels que le cyclohexanol, les cétones telles que l'acétone ou l'acétylacétone, les cyclocétones, telles que la cyclohexanone, le sulfolène ou leurs mélanges.

2. Procédé selon la revendication 1, **caractérisé en ce que** la réaction a lieu sous agitation.

3. Procédé selon la revendication 1 ou 2, **caractérisé en ce que** le composé de cuivre est choisi dans le groupe des composés de cuivre (II) constitué par le formiate, l'acétate, l'acétylacétonate, le sulfate, le bromure, le chlorure, le carbonate et le tartrate de cuivre (II), notamment sous forme anhydre.

4. Procédé selon l'une quelconque des revendications 1 à 3, **caractérisé en ce que** ledit au moins un composé organique au moins bidentate est choisi dans le groupe constitué par l'acide 1,2,3-benzène-tricarboxylique, l'acide 1,2,4-benzène-tricarboxylique et l'acide 1,3,5-benzène-tricarboxylique.

5. Procédé selon l'une quelconque des revendications 1 à 4, **caractérisé en ce que** le solvant non aqueux est choisi dans le groupe constitué par les alkylène polyols, les polyalkylène polyols, le DMF, le DEF et la NMP.

6. Procédé selon l'une quelconque des revendications 1 à 5, **caractérisé en ce que** le solvant non aqueux est l'éthylène glycol.

7. Procédé selon l'une quelconque des revendications 1 à 6, **caractérisé en ce que** la phase liquide pendant la réaction présente une teneur en eau maximale de 10 % en poids.

8. Procédé selon l'une quelconque des revendications 1 à 6, **caractérisé en ce que** de l'eau est extraite de la phase liquide pendant la réaction.

9. Procédé selon l'une quelconque des revendications 1 à 8, **caractérisé en ce qu'**après la réaction, le matériau de squelette métallo-organique formé est traité avec un autre solvant organique, qui présente un point d'ébullition plus bas que le solvant organique non aqueux utilisé lors de la réaction, afin d'éliminer le composé au moins bidentate ou le solvant organique non aqueux de la réaction éventuellement présent dans les pores du matériau de squelette métallo-organique.

10. Matériau de squelette métallo-organique poreux pouvant être obtenu par un procédé selon l'une quelconque des revendications 1 à 9.

11. Matériau de squelette selon la revendication 10, **caractérisé en ce que** celui-ci présente sous la forme de poudre une surface spécifique selon Langmuir de plus de 1 500 m²/g.

12. Utilisation d'un matériau de squelette selon la revendication 10 ou 11 pour l'absorption d'au moins une substance pour son stockage, sa séparation, sa libération contrôlée ou sa réaction chimique, ainsi qu'en tant que matériau support.
